# EUROPEAN PATENT APPLICATION

(11) **EP 2 258 336 A1**
(43) Date of publication of application: **08.12.2010**
(21) Application number: 10005369.3
(22) Date of filing: 21.05.2010
(51) Int. Cl.: A61K 6/00

(54) **Dental primer and dental adhesive set**

(30) Priority: 05.06.2009 JP 2009136374
(71) Applicant: GC Corporation, Itabashi-ku Tokyo 174-8585 (JP)
(72) Inventor: Arita, Akishi, Tokyo 174-8585 (JP); Hotta, Sayako, Tokyo 174-8585 (JP); Matsumoto, Naofumi, Tokyo 174-8585 (JP)
(74) Representative: Müller-Boré & Partner Patentanwälte

(57) **Abstract**

To provide a dental primer previously applied for strongly bonding a dental restoration made of various kinds of materials to dentine in a dental treatment field, and a dental adhesive set comprised of the dental primer and a dental adhesive having high dentine adhesiveness when used with the dental primer, the dental primer includes (meth)acrylate having an acid group, water, a water-soluble volatile organic solvent, and a vanadium compound, and can further include a filler and (meth)acrylate not having an acid group, while the dental adhesive set is comprised of the dental primer and a dental adhesive which includes (meth) acrylate having an acid group, (meth) acrylate not having an acid group, hydroperoxide as a polymerization initiator, a thiourea derivative as a reducing agent, and a vanadium compound as a polymerization accelerator, and can further include a filler.

## Description

The present invention relates to a dental primer used for previously applying in order to strongly bond a dental restoration made of a composite resin, metal, ceramics, or the like to dentine in a dental treatment field. Further, the present invention relates to a dental adhesive set comprised of the dental primer and a dental adhesive which can have high adhesiveness to dentine when used in combination with the dental primer.

In order to restore a tooth, a method, in which a dental restoration made of a composite resin, metal, ceramics, or the like is bonded to dentine with a dental adhesive, is widely performed. As for the dental adhesive for bonding the dental restoration to dentine, there are a light-cured adhesive, a self-cured adhesive, and a dual-cure type adhesive combining the light-cured adhesive and the self-cured adhesive, and these are chosen depending on kinds of dental restoration materials. In a restoration treatment in which a light-cured composite resin is directly filled in a tooth cavity, a dental adhesive called a light-cured bonding material is used. In a restoration treatment in which a dental restoration material made of a metal casting or ceramics is bonded to a tooth, a dental adhesive called a self-cured resin cement or a dual-cure type resin cement is used.

The dental adhesive is required to have property for strongly bonding a dental restoration to dentine for a long period of time even in such an environment as in an oral cavity. Thus, the dental adhesive needs to have sufficient dentine adhesiveness and adhesive durability. When the adhesive durability is poor, the dental restoration may drop, or a gap between the dentine and the dental restoration may be created, and bacterial intruded in the gap may cause a secondary caries.

Conventionally, a method of pre-treating a tooth surface before applying the dental adhesive has been used in order to improve the dentine adhesiveness. A general pretreatment material is an acid solution decalcificating the tooth surface, and the acid solution such as a phosphoric acid solution, a maleic acid solution, a citric acid solution and the like have been used. Since the dental adhesive hardly permeates an exposing collagen layer, a dental adhesive permeation accelerator called a dental primer is used in addition. However, since applications in use of the dental primer for enamel and dentine are different from each other, the treatment becomes to be complicated.

For example, Japanese Patent Application Laid-Open No. 3-240712, Japanese Patent Application Laid-Open No. 4-8368, Japanese Patent Application Laid-Open No. 6-192029, Japanese Patent Application Laid-Open No. 6-192030, Japanese Patent Application Laid-Open No. 7-82115, Japanese Patent Application Laid-Open No. 7-89820, and Japanese Patent Application Laid-Open No. 7-97306 discuss dental primers capable of simultaneously performing both the pretreatments of enamel and dentine. Any of the dental primers can be accurately photopolymerized when a light-cured dental adhesive such as a bonding material or the like for a composite resin is used, and can give sufficient bond strength to both the enamel and dentine. However, when a self-cured dental adhesive such as a resin cement or the like, or a dual-cure type dental adhesive is used, the dental primers cannot give sufficient dental adhesiveness. Particularly, the self-cured dental adhesive of two components mixing type generally has a lower polymerization rate in comparison with the light-cured dental adhesive, and thus causes the problem of insufficient dentine adhesiveness. In order to obtain high dentine adhesiveness, there are the self-cured dental adhesives in which trialkylboron (or partially oxidized trialkylboron) having high catalyst activity is used. However, this catalyst needs to be dividedly packaged because of being chemically unstable. Further, since handling of the catalyst is hard, the application becomes to be more complicated. Therefore, it has been required to develop a pretreatment material (a dental primer) which can produce excellent dentine bond strength when not only using the light-cured dental adhesive but also using the self-cured dental adhesive or dual-cure type dental adhesive, and to develop a dental adhesive set which can exercise high effectiveness in combination with such the dental primer.

The present invention is directed to a dental primer capable of giving high adhesiveness to both dentine and enamel with only one application, regardless of a self-cured dental adhesive, dual-cure type dental adhesive, or light-cured dental adhesive. Further, the present invention is directed to a dental adhesive set comprised of the dental primer and a dental adhesive having high dentine adhesiveness when used with the dental primer.

Present inventors carried out earnest works to solve the aforementioned problems, and as a result, they found out the followings to complete the present invention. A dental primer capable of giving sufficient dentine adhesiveness even through chemical polymerization only can be produced by adding a vanadium compound to a composite including (meth) acrylate having an acid group, water, and a water-soluble volatile organic solvent. Further, the dental primer can include a filler and (meth)acrylate not having an acid group. Furthermore, high dentine adhesiveness can be obtained by using the aforementioned dental primer with a dental adhesive which includes (meth) acrylate having an acid group, (meth) acrylate not having an acid group, hydroperoxide as a polymerization initiator, a thiourea derivative as a reducing agent, and a vanadium compound as a polymerization accelerator. In addition, the dental adhesive can also include a filler.

That is, a dental primer according to an aspect of the present invention includes (meth) acrylate having an acid group, water, a water-soluble volatile organic solvent, and a vanadium compound, and can further include a filler and (meth)acrylate not having an acid group. A dental adhesive set according to an aspect of the present invention is comprised of the aforementioned dental primer and a dental adhesive which includes (meth)acrylate having an acid group, (meth) acrylate not having an acid group, hydroperoxide as a polymerization initiator, a thiourea derivative as a reducing agent, and a vanadium compound as a polymerization accelerator, and can also include a filler in addition.

More particularly, the dental primer includes 3 to 35% by weight of (meth) acrylate having an acid group, 5 to 70% by weight of water, 20 to 80% by weight of a water-soluble volatile organic solvent, and 0.01 to 1% by weight of a vanadium compound, and can further include 0.5 to 50% by weight of a filler, and 5 to 25% by weight of (meth)acrylate not having an acid group. The dental adhesive set is comprised of the aforementioned dental primer and the dental adhesive which includes 7 to 60% by weight of (meth) acrylate not having an acid group, 3 to 30% by weight of (meth)acrylate having an acid group, 0.01 to 10% by weight of hydroperoxide as a polymerization initiator, 0.01 to 10% by weight of a thiourea derivative as a reducing agent, and 0.001 to 5% by weight of a vanadium compound, and can also include 0.5 to 80% by weight of a filler in addition.

The dental primer and the dental adhesive set according to the present invention are applied to a dentine surface, and can give high adhesiveness to both dentine and enamel with only one application, regardless of a self-cured adhesive, dual-cure type adhesive, or light-cured adhesive.

(Meth)acrylate of the present invention means various kinds of monomers, oligomers, and prepolymers of acrylate or methacrylate. (Meth)acrylate having an acid group has the effect for improving adhesiveness to dentine. The (meth)acrylate having an acid group is preferably (meth)acrylate having a phosphate group or a carboxyl group, and the (meth)acrylate having one or more phosphate groups or carboxyl groups in one molecule can be used. Since the phosphate group has acidity stronger than the carboxylic group, the (meth)acrylate having the phosphate group has the high effects for dissolving a smear layer of a tooth surface and for decalcificating dentine. Particularly, the (meth) acrylate having the phosphate group exercises the effect of highly improving adhesiveness to enamel.

The (meth)acrylate having a phosphate group can be 2-(meth)acryloyloxyethyldihydrogen phosphate, bis[2-(meth)acryloyloxyethyl]hydrogen phosphate, 2-(meth)acryloyloxyethylphenylhydrogen phosphate, 6-(meth)acryloyloxyhexyldihydrogen phosphate, 6-(meth)acryloyloxyhexylphenylhydrogen phosphate, 10-(meth)acryloyloxydecyldihydrogen phosphate, 1, 3-di(meth)acryloylpropane-2-dihydrogen phosphate, 1, 3-di(meth)acryloylpropane-2-phenylhydrogen phosphate, bis[5-{2-(meth)acryloyloxyethoxycarbonyl} heptyl] hydrogen phosphate, or the like. Particularly, 10-(meth)acryloyloxydecyldihydrogen phosphate is preferable because of having excellent adhesive property and stability of (meth)acrylate itself. These (meth)acrylates having the phosphate group can be used independently or by mixing two or more kinds.

The (meth)acrylate having the carboxyl group can be 4-(meth)acryloxyethyltrimellitic acid, 4-(meth)acryloxyethyltrimellitic acid anhydride, 4-(meth)acryloxydecyltrimellitic acid, 4-(meth)acryloxydecyltrimellitic acid anhydride, 11-(meth)acryloyloxy-1, 1-undecanedicarboxylic acid, 1, 4-di(meth)acryloyloxypyromellitic acid, 2-(meth)acryloyloxyethylmaleic acid, 2-(meth)acryloyloxyethylphthalic acid, 2-(meth)acryloyloxyethylhexahydrophthalic acid, or the like. Particularly, 4-(meth)acryloxyethyltrimellitic acid and 4-(meth)acryloxyethyltrimellitic acid anhydride are preferable because of having an excellent adhesive property.

The blending amount of the (meth)acrylate having an acid group to the dental primer is preferably 3 to 35% by weight, and the blending amount of the (meth)acrylate having an acid group to the dental adhesive is preferably 3 to 30% by weight. If the blending amount is less than 3% by weight, the adhesiveness to dentine tends to decrease. If the blending amount to the dental primer is more than 35% by weight or the blending amount to the dental adhesive is more than 30% by weight, the curability of the dental primer or the dental adhesive itself decreases and the bond strength decreases. In addition, the blending amount to the dental primer is preferably 30% by weight or less. Furthermore, when the dental primer is comprised of a plurality of components, the blending amount is regulated with the blending amount in a mixing ratio at a time of use.

Particularly, the (meth)acrylate not having an acid group can be methyl (meth)acrylate, ethyl (meth)acrylate, isopropyl (meth)acrylate, n-butyl (meth)acrylate, isobutyl (meth)acrylate, hydroxypropyl (meth)acrylate, tetrahydrofurfryl (meth)acrylate, glycidyl (meth)acrylate, 2-hydroxyethyl (meth)acrylate, 2-hydroxypropyl (meth)acrylate, 3-hydroxypropyl (meth)acrylate, 2-methoxyethyl (meth)acrylate, 2-ethoxyethyl (meth)acrylate, 2-ethylhexyl (meth)acrylate, benzyl (meth)acrylate, 2-hydroxy-1, 3-di(meth)acryloxy propane, ethylene glycol di(meth)acrylate, diethylene glycol di(meth)acrylate, triethylene glycol di(meth)acrylate, butylene glycol di(meth)acrylate, neopentyl glycol di(meth)acrylate, 1, 3-butanediol di(meth)acrylate, 1, 4-butanediol di(meth)acrylate, 1, 6-hexanediol di(meth)acrylate, trimethylolpropane tri(meth)acrylate, trimethylolethane tri(meth)acrylate, pentaerythritol tri(meth)acrylate, trimethylolmethane tri(meth)acrylate, pentaerythritol tetra(meth)acrylate, polybutylene glycol di(meth)acrylate, or bisphenol A glycidyl (meth)acrylate. A monomer, oligomer, or prepolymer of these can be preferably used. Further, as for (meth)acrylate having urethane bond, di-2-(meth)acryloxyethyl-2, 2, 4-trimethylhexamethylene dicarbamate, 1, 3, 5-tris [1, 3-bis{(meth)acryloyloxy}-2-propoxycarbonylaminohexa ne]-1, 3, 5-(1H, 3H, 5H) triazine-2, 4, 6- trione, and 2, 2-bis-4-(3-(meth)acryloxy-2-hydroxypropyl)-phenylpropane, or the like can be used. In addition, the (meth)acrylate having urethane bond can be (meth)acrylate of urethane oligomer comprised of 2, 2'-di(4-hydroxycyclohexyl) propane, 2-oxypanone, hexamethylene diisocyanate, and 2-hydroxyethyl (meth)acrylate, and (meth)acrylate of urethane oligomer comprised of 1, 3-butanediol, hexamethylene diisocyanate, and 2-hydroxyethyl (meth)acrylate, and the like. These can be used independently or by mixing two or more kinds.

The blending amount of the (meth)acrylate not having an acid group to the dental primer is preferably 5 to 25% by weight, and the blending amount of the (meth)acrylate not having an acid group to the dental adhesive is preferably 7 to 60% by weight. The dental primer does not need to include the (meth)acrylate not having an acid group. The reason of blending the (meth)acrylate having an acid group to the dental adhesive is for improving adhesiveness to ceramics such as zirconia, alumina and the like or alloys including noble metals, which are materials for a dental restoration. If the blending amount of the (meth)acrylate having an acid group to the dental adhesive is less than 7% by weight, sufficient adhesiveness cannot be obtained. If the blending amount is more than 60% by weight, curability of the dental adhesive tends to decrease.

By blending water to the dental primer together with the aforementioned (meth)acrylate having an acid group, the acid group of the (meth)acrylate having the acid group is dissociated, and acts as a self-etching primer. Thus, the dissociated acid group has the effects for dissolving a smear layer on a tooth surface, decalcifying dentine, and improving permeability to dentine. Therefore, the dental primer can be used without performing the pretreatment with a phosphoric acid etching solution or the like. Further, when the pretreatment with the phosphoric acid etching solution or the like is performed in advance, permeability to dentine is more improved, and the bond strength is improved. The blending amount of water is preferably 5 to 70% by weight. If the blending amount is less than 5% by weight, ability for decalcifying dentine and the permeability decrease. If the blending amount is more than 70% by weight, the dental primer is hard to be equalized.

The vanadium compound is a polymerization accelerator for redox polymerization, and acts as a polymerization catalyst together with the reducing agent in the dental adhesive which is used after using the dental primer. The vanadium compound is a comparatively stable material in (meth) acrylate. Thus, a one-liquid type dental primer can be produced by blending the vanadium compound with the (meth) acrylate. Preferably, the blending amount of the vanadium compound to the dental primer and the dental adhesive of the dental adhesive set is 0.001 to 1% by weight. If the blending amount is less than 0.001% by weight, the effect as the polymerization accelerator tends to be insufficient. If the blending amount is more than 1% by weight, the dental primer is colored to dark green, and the (meth)acrylate having an acid group may be polymerized while the dental primer is stored such being disadvantageous. The vanadium compound can be vanadium acetylacetonate, vanadyl acetylacetonate, vanadyl stearate, vanadium naphthenate, vanadium benzoyl acetonate, or the like. Particularly, one or more kinds selected from vanadium acetylacetonate and vanadyl acetylacetonate are preferable.

In addition to the vanadium compound, the dental primer and the dental adhesive can include sulfinic acid or its salt as a polymerization accelerator according to necessity. More particularly, the sulfinic acid or its salt can be benzenesulfinic acid, benzenesulfinic acid sodium, p-toluenesulfinic acid, p-toluenesulfinic acid sodium, 2,4,6-trimethylbenzenesulfinic acid, 2,4,6-trimethylbenzenesulfinic acid sodium, 2,4,6-triethylbenzenesulfinic acid, 2,4,6-triethylbenzenesulfinic acid sodium, 2,4,6-triisopropylbenzenesulfinic acid, 2,4,6-triisopropylbenzenesulfinic acid sodium, or the like. In these compound, benzenesulfinic acid sodium, p-toluenesulfinic acid sodium, and 2,4,6-triisopropylbenzenesulfinic acid sodium are preferable from the viewpoints of preservation stability.

The dental primer includes a water-soluble volatile organic solvent in order to give compatibility to the composites constituting the primer and permeate the (meth) acrylate having an acid group in dentine. The organic solvent can be acetone, ethanol, methanol, methylethyl ketone, isopropanol, or the like. The water-soluble volatile organic solvent can be used independently or by mixing two or more kinds. In these solvents, acetone and ethanol are particularly preferable. The blending amount of the organic solvent to the dental primer is preferably 20 to 80% by weight. If the blending amount is less than 20% by weight or more than 80% by weight, sufficient bond strength cannot be obtained.

The dental primer according to the present invention can further include a filler to adjust viscosity. The filler can be powders of anhydrous silicic acid, glasses such as barium glass, strontium glass, alumina glass, potassium glass, fluoroaluminosilicate glass and the like, synthetic zeolite, calcium phosphate, feldspar, fumed silica, aluminum silicate, calcium silicate, magnesium carbonate, hydrous silicic acid, hydrous calcium silicate, hydrous aluminum silicate, quartz, or the like. In order to bond with (meth) acrylate, the filler can be subjected to a surface treatment with a silane coupling agent such as γ-methacryloxypropyltrimethoxysilane, vinyltrichlorosilane, vinyltriethoxysilane, vinyltrimethoxysilane, vinyltriacetoxysilane, vinyltri (methoxyethoxy) silane, or the like. Further, an organic/inorganic composite filler can be used, where the organic/inorganic composite filler is produced by previously mixing the filler with a monomer or an oligomer, curing the mixture, and pulverizing the cured body. These fillers can be used independently or by mixing two or more kinds. In these fillers, anhydrous silicic acid, hydrous silicic acid, hydrous calcium silicate and hydrous aluminum silicate have an effect to prevent the dental adhesive before polymerization from gelling even when the dental adhesive is stored for a long period of time. The blending amount of the filler to the dental primer is preferably 0.5 to 50% by weight. If the blending amount is less than 0.5% by weight, a blending effect of the filler cannot be obtained. If the blending amount is more than 50% by weight, the dental primer is hardly applied on a tooth surface, and operativity becomes to be hard.

Packages of the dental primer and the dental adhesive according to the present invention are not restricted especially, and can be properly decided considering preservation stability. For example, when the dental primer or the dental adhesive includes a component which comes to be a polymerization catalyst of (meth)acrylate by itself, such as sulfinic acid or its salt, the polymerization catalyst and (meth)acrylate are divided into two with an arbitrary combination, and are mixed just before using those. However, one liquid is preferable, considering operativity.

The dental primer according to the present invention can be used with conventional dental adhesives, such as a dental bonding material, a resin cement, and the like. Particularly, more excellent adhesiveness to a dentin can be obtained when a dental adhesive in a dental adhesive set according to the present invention is used, where the dental adhesive includes (meth)acrylate having an acid group, (meth) acrylate not having an acid group, hydroperoxide as a polymerization initiator, a thiourea derivative as a reducing agent, and a vanadium compound as a polymerization accelerator. Therefore, another aspect of the present invention is a dental adhesive set comprised of a dental primer and a dental adhesive, where the dental primer includes (meth)acrylate having an acid group, water, a water-soluble volatile organic solvent, and a vanadium compound, and can further include a filler and (meth)acrylate not having an acid group, and the dental adhesive includes (meth) acrylate having an acid group, (meth) acrylate not having an acid group, hydroperoxide as a polymerization initiator, a thiourea derivative as a reducing agent, and a vanadium compound as a polymerization accelerator.

As for a polymerization reaction used for the dental adhesive set according to the present invention, an oxidizing and reducing reaction of the hydroperoxide as a polymerization initiator and the thiourea derivative as a reducing agent, which are in the dental adhesive, is used with the vanadium compound in the dental primer and the dental adhesive.

The blending amount of the hydroperoxide to the dental adhesive is preferably 0.01 to 10% by weight. If the blending amount is less than 0.01% by weight, a function as a redox polymerization initiator tends to be insufficient. If the blending amount is more than 10% by weight, (meth)acrylate in the dental adhesive tends to be polymerized easily, and preservation stability of the composition decreases. For example, the hydroperoxide can be cumene hydroperoxide, t-butyl hydroperoxide, t-amyl hydroperoxide, 1,1,3,3-tetramethylbutyl hydroperoxide, 2,5-dimethyl-2,5-di(hydroperoxy)hexane, p-diisopropylbenzene monohydroperoxide, p-methane hydroperoxide, or pinane hydroperoxide. Particularly, cumene hydroperoxide and t-butyl hydroperoxide are preferable from the viewpoints of polymerization ability and preservation stability.

The thiourea derivative is a reducing material for redox polymerization, and is a stable material in (meth)acrylate. The content of the thiourea derivative in the dental adhesive is preferably 0.01 to 10% by weight. If the content is less than 0.01% by weight, an ability as a polymerization catalyst is insufficient. If the content is more than 10% by weight, the thiourea derivative may not be dissolved with (meth)acrylate. The thiourea derivative is preferably one or more kinds selected from ethylenethiourea, diethylthiourea, tetramethylthiourea, N-acetylthiourea, N-benzoylthiourea, diphenylthiourea, and dicyclohexylthiourea. Particularly, N-acetylthiourea and N-benzoylthiourea are preferable in the view point of polymerization ability.

The dental adhesive in the dental adhesive set according to the present invention can include a filler in order to adjust viscosity and increase the strength of the cured composition. The filler can be powders of anhydrous silicic acid, glasses such as barium glass, strontium glass, alumina glass, potassium glass, fluoroaluminosilicate glass and the like, synthetic zeolite, calcium phosphate, feldspar, fumed silica, aluminum silicate, calcium silicate, magnesium carbonate, hydrous silicic acid, hydrous calcium silicate, hydrous aluminum silicate, quartz, or the like. In order to bond with (meth)acrylate, these fillers can be subjected to a surface treatment with a silane coupling agent such as γ-methacryloxypropyltrimethoxysilane, vinyltrichlorosilane, vinyltriethoxysilane, vinyltrimethoxysilane, vinyltriacetoxysilane, vinyltri (methoxyethoxy) silane, or the like. Further, an organic/inorganic composite filler can be used, where the organic/inorganic composite filler is produced by previously mixing the filler with a monomer or an oligomer, curing the mixture, and pulverizing the cured body. The filler can be used independently or by mixing two or more kinds. In these fillers, anhydrous silicic acid, hydrous silicic acid, hydrous calcium silicate, and hydrous aluminum silicate have an effect to prevent the dental adhesive before polymerization from gelling even when the dental adhesive is stored for a long period of time. The blending ratio of the filler to the dental primer is preferably 0.5 to 80% by weight. If the blending ratio is less than 0.5% by weight, a blending effect of the filler cannot be obtained. If the blending ratio is more than 80% by weight, the dental adhesive is hardly applied on a tooth surface, and operativity becomes to be hard.

A method for using the dental primer and the dental adhesive set according to the present invention will be described. The dental primer is firstly applied on a tooth surface with a brush or the like, and dried with air. At this time, the tooth surface can be pretreated with a phosphoric acid etching solution or the like in advance. When the dental primer is comprised of a plurality of components, the plurality of components of the dental primer is mixed before applying of the dental primer. As for a mixing method of the dental adhesive, a technician can manually mix it using a spatula and a mixing paper, or can mix it using an auto mixing system with a mixing tip. Then, the dental adhesive is applied on the tooth surface and the inner surface of a dental prosthesis, and the dental prosthesis is applied to a portion to be restored, and is pressed to and fixed at this portion. When the dental prosthesis is produced from a material and in a shape having appreciable light transmittance, a dual-cure type dental adhesive can be used.

The polymerizable composition according to the present invention can suitably include a photopolymerization catalyst, a polymerization inhibitor, an antibacterial agent, a fluorescent agent, a pigment, and the like, which are conventionally used according to necessity.

### [Example]

Dental primers were produced with blending ratios (% by weight) illustrated in Tables 1 to 4. The dental primers were combined with a commercial dental adhesive and dental adhesives A to F produced with blending ratios (% by weight) illustrated in Table 5, and subjected to a tensile bond strength test. In addition, as a conventional dental primer not including a vanadium compound, a commercial dental primer (GC LINKMAX SELF-ETCHING PRIMER, produced by GC Corporation) was used. As a conventional resin cement using a tert-amine-based polymerization catalyst and conventional benzoyl peroxide not including a vanadium compound, a commercial resin cement (GC LINKMAX, produced by GC Corporation) was used.

Brevity codes in the tables are as follows.
GDMA: 2-hydroxy-1, 3-dimethacryloxy propane
TEGDMA: Triethylene glycol dimethacrylate
UDMA: Di-2-methacryloxyethyl-2, 2, 4-trimethylhexamethylene dicarbamate
Bis-GMA: 2,2-bis-4-(3-methacryloyloxy-2-hydroxypropyl)-phenylpropane
MDP: 10-methacryloyloxydecyldihydrogen phosphate
4-MET: 4-methacryloxyethyltrimellitic acid
t-BHP: tert-butyl hydroperoxide
CHP: Cumene hydroperoxide
NATU: N-acetylthiourea
NBTU: N-benzoylthiourea
SiO2: Silicon dioxide
Aerosil: Fumed silica (R812, produced by Nippon Aerosil Co., Ltd.) VO(C5H702)2: Vanadyl acetylacetonate
V(C5H702)3: Vanadium acetylacetonate
pTSNa: p-toluenesulfinic acid sodium
BSNa: Benzenesulfinic acid sodium
IA: 6-tert-butyl-2,4-xylenol
EPA: p-ethyl dimethylaminobenzoate
CQ: Photopolymerization initiator (Camphorquinone)
Polymerization inhibitor: 2,6-di-tert-butyl-p-cresol

### [Tensile bond strength test 1]

The labial surface of a bovine tooth crown part was polished by a waterproof polishing paper of #320, while injecting water thereto, so as to expose enamel or dentine. A tape made of Teflon having a hole with a diameter of 2.5mm was attached to a surface to be adhered so that an adhesion area was prescribed. The dental primers in Tables 1 and 2 were applied on the tooth surface of which the area was prescribed (after mixing two liquids in case of the dental primer in Table 2), and dried with air after 20 seconds. Then, two pastes of dental adhesives in Table 5 were mixed at the weight ratio of 1:1, and the mixed dental adhesive was applied on the dried dental primers and pressed by a stainless rod. At this time of the tensile bond strength test, polymerization was made by only a chemical polymerization, without irradiating light at all. A tested specimen was stored for 24 hours in water at 37°C. Then, the tested specimen was subjected to a tensile bond strength test by a tensile tester (AUTOGRAPH, produced by SHIMADZU CORPORATION) at a crosshead speed of 1 mm/min.

### [Tensile bond strength test 2]

Enamel or dentine of a bovine tooth was exposed by a method similar to the tensile bond strength test 1. Before using a dental primer, the bovine tooth was etched for 10 seconds using a tooth surface treating agent which is a phosphoric acid etching solution (GC LINK MASTER ETCHANT, produced by GC Corporation), washed with water, and dried. Then, a tape made of Teflon having a hole with a diameter of 2.5mm was attached to a surface to be adhered so that an adhesion area was prescribed. Dental primers in Tables 3 and 4 were applied on a tooth surface of which the area was prescribed (after mixing two liquids at a weight ratio of 1:1 in case of the dental primer in Table 4), and dried with air after 20 seconds. Then, a tensile bond strength test 2 was performed using the dental adhesive in Table 5 by a method similar to the tensile bond strength test 1.

**[Table 1]**

| (% by weight) | | | Example 1 | Example 2 | Example 3 | Example 4 | Example 5 | Example 6 | Example 7 | Example 8 | Example 9 | Comparative example 1 | Comparative example 2 | Comparative example 3 | Comparative example 4 |
|---|---|---|---|---|---|---|---|---|---|---|---|---|---|---|---|
| | (Meth)acrylate not having an acid group | GDMA | 15 | 15 | 7.5 | 5 | | | 3 | | | 15 | 5 | | |
| | | TEGDMA | | | | 2.5 | 7.5 | 7.5 | | 7.5 | 7.5 | | 2.5 | 14.6 | 7.5 |
| | | UDMA | | | | 2.5 | 2.5 | 2.5 | | 2.5 | 2.5 | | 2.5 | 4.9 | 2.5 |
| | | Bis-GMA | 5 | 5 | 2.5 | | | | 1 | | | 5 | | | |
| Dental Primer | (Meth)acrylate having an acid group | MDP | 9.5 | 9.5 | 4.75 | 9.5 | 9.5 | 4.75 | 17.5 | | 4.75 | 9.5 | 9.5 | | 4.75 |
| | | 4-MET | 9.5 | 9.5 | 4.75 | | | 4.75 | 17.5 | 9.5 | 4.75 | 9.5 | | | 4.75 |
| | Vanadium compound | VO(C₅H₇O₂)₂ | 0.4 | 0.4 | 0.25 | 0.25 | | | 0.4 | | | | | | |
| | | V(C₅H₇O₂)₃ | | | | | 0.25 | 0.25 | | 0.25 | 0.25 | | | 0.25 | 0.25 |
| | Organic solvent | Acetone | 51.05 | 46.05 | 60.7 | 55.7 | 70.7 | 65.7 | 46.06 | 60.7 | 55.7 | 46.05 | 55.95 | 55.7 | 80.2 |
| | | Water | 9.5 | 14.5 | 19.5 | 24.5 | 9.5 | 14.5 | 14.5 | 19.5 | 24.5 | 14.9 | 24.5 | 24.5 | |
| | Other component | Polymerization inhibitor | 0.05 | 0.05 | 0.05 | 0.05 | 0.05 | 0.05 | 0.05 | 0.05 | 0.05 | 0.05 | 0.05 | 0.05 | 0.05 |
| | Total | | 100 | 100 | 100 | 100 | 100 | 100 | 100 | 100 | 100 | 100 | 100 | 100 | 100 |
| Dental Adhesive | | | A | A | A | A | B | B | B | B | A | A | A | B | B |
| Tensile adhesive strength (Mpa) | | To enamel | 24.1 | 26.3 | 26.3 | 29.3 | 25.9 | 25.2 | 23.2 | 19.3 | 28.5 | 7.4 | 5.1 | 6.4 | 7.8 |
| | | To dentine | 25.6 | 26.1 | 25.3 | 21.4 | 20.7 | 25.5 | 24.9 | 11.4 | 25.5 | 5.4 | 5 | 6.2 | 9.6 |

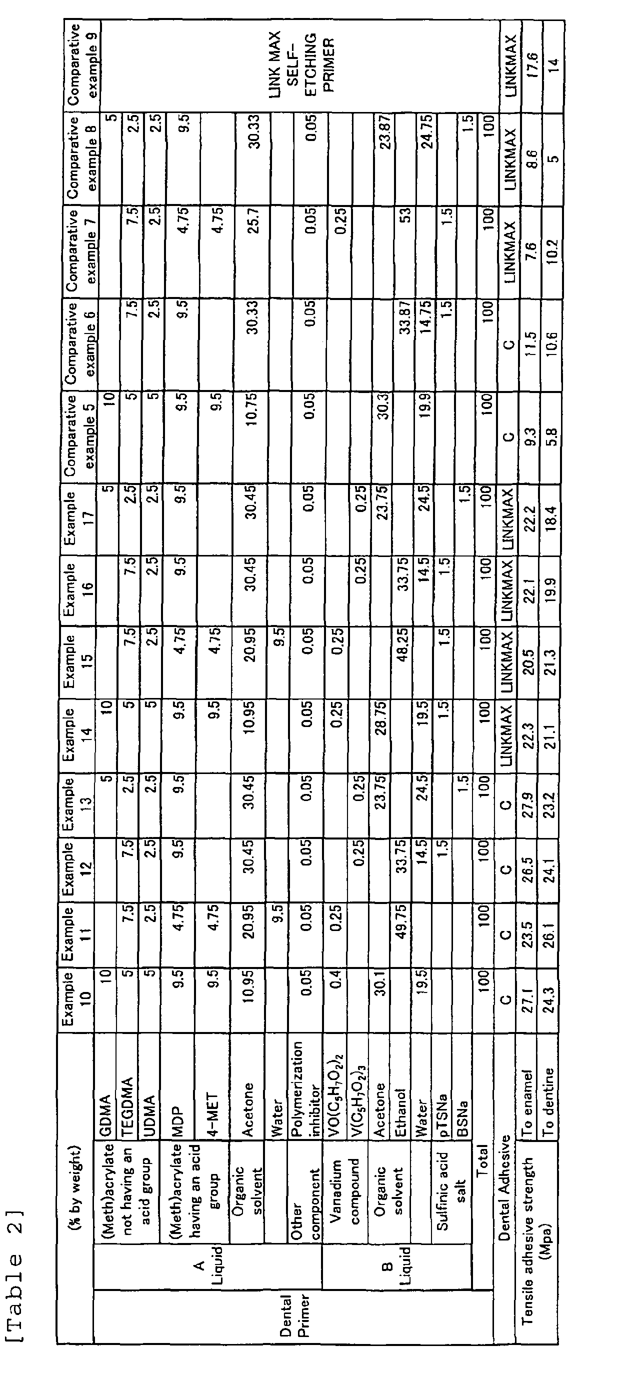

**[Table 3]**

| (% by weight) | | | Example 18 | Example 19 | Example 20 | Example 21 | Example 22 | Example 23 | Example 24 | Comparative example 10 | Comparative example 11 | Comparative example 12 | Comparative example 13 |
|---|---|---|---|---|---|---|---|---|---|---|---|---|---|
| | (Meth)acrylate TEGDMA not having an acid group | GDMA | 15 | 15 | 5 | | 3 | | | 15 | 5 | | |
| | | | | | | 2.5 7.5 | | 7.5 | 7.5 | | 25 | 14.6 | 7.5 |
| | | UDMA | | | 2.5 | 2.5 | | 2.5 | 2.5 | | 2.5 | 4.9 | 2.5 |
| | | Bis-GMA | 5 | 5 | | | 1 | | | 5 | | | |
| | (Meth)acrylate having an acid group | MDP | 9.5 | 9.5 | 4.75 | 9.5 | 35 | 4.75 | | 9.5 | 4.75 | | 4.75 |
| Dental Primer | | 4-MET | 9.5 | 9.5 | 4.75 | | | 4.75 | 9.5 | 9.5 | 4.75 | | 4.75 |
| | Vanadium | VO(C₅H₇O₂)₂ | 0.4 | 0.4 | 0.25 | | 0.4 | | | | | | |
| | compound | V(C₅H₇O₂)₃ | | | | 0.25 | | 0.25 | 0.25 | | | 0.25 | 0.25 |
| | Organic solvent | Acetone | 51.05 | 46.05 | 60.7 | 70.7 | 46.05 | 65.7 | 60.7 | 51.05 | 60.7 | 65.7 | 80.2 |
| | | Water | 9.5 | 14.5 | 19.5 | 9.5 | 14.5 | 14.5 | 19.5 | 9.9 | 19.75 | 14.5 | |
| | Other component | Polymerization inhibitor | 0.05 | 0.05 | 0.05 | 0.05 | 0.05 | 0.05 | 0.05 | 0.05 | 0.05 | 0.05 | 0.05 |
| | Total | | 100 | 100 | 100 | 100 | 100 | 100 | 100 | 100 | 100 | 100 | 100 |
| Dental Adhesive | | | D | D | D | E | E | E | D | D | D | E | E |
| Tensile adhesive strength (Mpa) | | To enamel | 29.6 | 30.1 | 31.4 | 32.3 | 31.7 | 19.2 | 24.8 | 13.9 | 14.6 | 17.5 | 21.6 |
| | | To dentine | 26.9 | 28.7 | 30.1 | 25.4 | 25.8 | 11.9 | 28.7 | 6.7 | 5.5 | 7.2 | 13.2 |

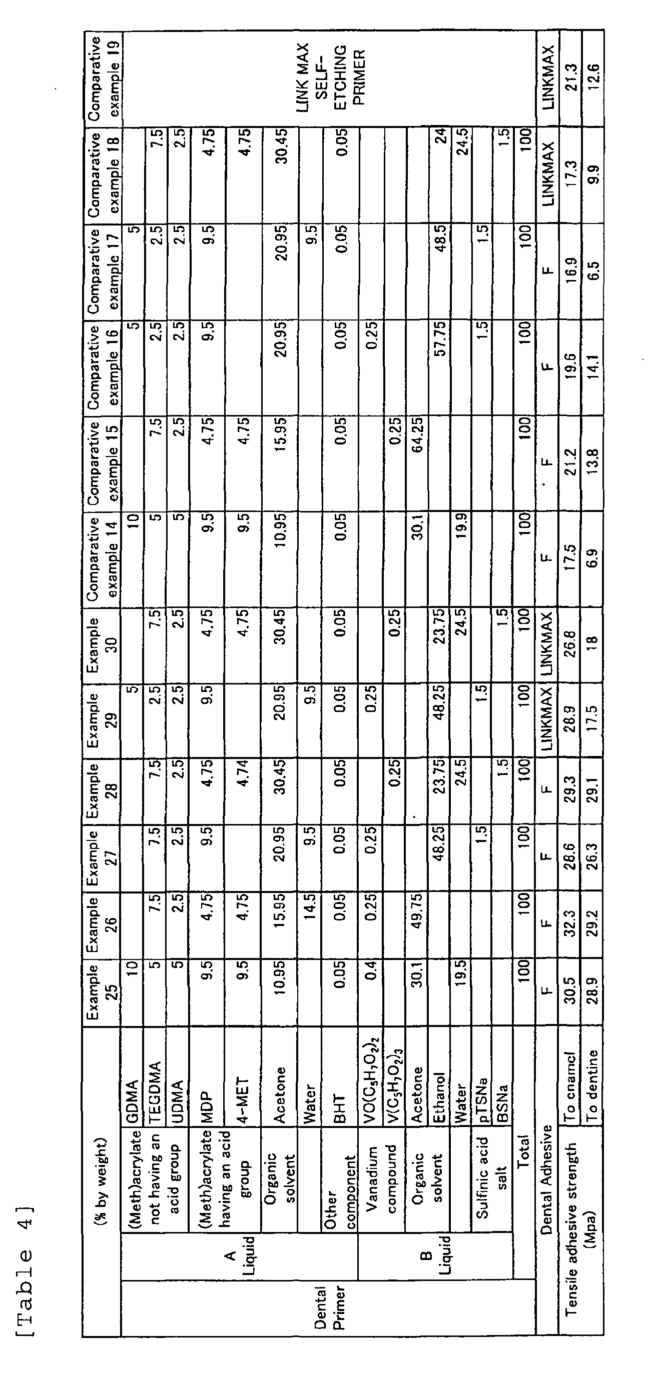

**[Table 5]**

| (% by weight) | | | Adhesive A | Adhesive B | Adhesive C | Adhesive D | Adhesive E | Adhesive F |
|---|---|---|---|---|---|---|---|---|
| | (Meth)acrylate not having an acid group | Bis-GMA | | | | | | 16.66 |
| | | TEGDMA | | | | | 26.1 | 14 |
| | | UDMA | 50.11 | 63.25 | 37.5 | 15.5 | 50.11 | |
| | | GDMA | 26.1 | 13.25 | 7.5 | 10.5 | | |
| | (Meth)acrylate having an acid group | MDP | 8.7 | 8.7 | 5 | | 8.7 | 3.5 |
| | | 4-META | | | | 5 | | |
| A Paste | Hydroperoxide CHP | t-BHP | 2 | 2 | 0.8 | | | |
| | | | | | | 2 | 2 | 0.8 |
| | Filler | SiO2 Powder | | | 47.3 | 60.96 | | 62.5 |
| | | Aerosil | 13 | 12.71 | 1.85 | 3 | 13 | 2.5 |
| | Other additive | Water | | | | 3 | | |
| | | IA | 0.09 | 0.09 | 0.05 | 0.04 | 0.09 | 0.04 |
| Total | | | 100 | 100 | 100 | 100 | 100 | 100 |
| | (Meth)acrylate not having an acid group | Bis-GMA | | | | | 14 | 18.6 |
| | | TEGDMA | | 6.25 | | 9 | 14 | |
| | | UDMA | 12.5 | 12.5 | 29.6 | 21 | | |
| | | GDMA | 12.5 | 6.25 | 7.4 | | | 27.9 |
| | Thiourea derivative | NATU | 0.25 | 0.38 | 0.52 | | | |
| | | NBTU | | | | 0.3 | 0.42 | 0.7 |
| | Vandadium compound | VO(C₅H₇O₂)₂ | 0.02 | 0.03 | 0.04 | | | |
| B Paste | | V(C₅H₇O₂)₃ | | | | 0.03 | 0.03 | 0.03 |
| | Filler | Si02 Powder | | | 60 | | | |
| | | Aerosil | 3.7 | 3.7 | 2 | 4.31 | 1 | 2.22 |
| | | Fluoroaluminosilicate glass powder | 70.73 | 70,6 | | 65 | | |
| | | Strontium glass powder | | | | | 70.21 | 50 |
| | Other additive | EPA | 0.25 | 0.25 | 0.37 | 0.3 | 0.28 | 0.45 |
| | | CQ | 0.02 | 0.02 | 0.03 | 0.03 | 0.02 | 0.05 |
| | | IA | 0.03 | 0.02 | 0.04 | 0.03 | 0.04 | 0.05 |
| Total | | | 100 | 100 | 100 | 100 | 100 | 100 |

As clearly seen from Tables 1 to 4, it can be confirmed on the basis of the results in Tables 3 and 4, where a tooth surface was pre-treated with a phosphoric acid etching solution, and Tables 1 and 2, where a tooth surface was not pre-treated, that the dental primers according to the present invention have very high tensile bond strength even though a chemical polymerization only, and a convention technique could not have such the high tensile bond strength. Particularly, the maximum bond strength could be obtained by combining the dental primer with the dental adhesive including (meth) acrylate having an acid group, (meth) acrylate not having an acid group, hydroperoxide as a polymerization initiator, a thiourea derivative as a reducing agent, and a vanadium compound as a polymerization accelerator, while it could be conformed that the high bond strength could also be obtained by combining the dental primer with a resin cement or the like which was conventionally used.

As clearly seen from each of comparative examples, when the dental primer did not include the vanadium compound (Comparative examples 1, 2, 5, 6, 8, 9, 10, 11, 14, 17, 18, 19), and when the dental primer did not include the combination of water and (meth)acrylate having an acid group (Comparative examples 3, 4, 7, 12, 13, 15, 16), the bond strength was extremely low.

## Claims

1. A dental primer comprising (meth)acrylate having an acid group, water, a water-soluble volatile organic solvent, and a vanadium compound.

2. The dental primer as claimed in claim 1, wherein the dental primer comprises 3 to 35% by weight of the (meth) acrylate having an acid group, 5 to 70% by weight of the water, 20 to 80% by weight of the water-soluble volatile organic solvent, and 0.001 to 1% by weight of the vanadium compound.

3. The dental primer as claimed in claim 1 or 2, wherein the dental primer further comprises a filler and/or (meth)acrylate not having an acid group.

4. The dental primer as claimed in claim 3, wherein a blending ratio of the filler is 0.5 to 50% by weight, and a blending ratio of the (meth)acrylate not having an acid group is 5 to 25% by weight.

5. The dental primer as claimed in any one of claims 1 to 4, wherein the vanadium compound is one or more kinds selected from vanadium acetylacetonate, vanadyl acetylacetonate, vanadyl stearate, vanadium naphthenate, and vanadium benzoyl acetonate.

6. A dental adhesive set comprising:
the dental primer as claimed in any one of claims 1 to 5; and
a dental adhesive comprised.of (meth)acrylate having an acid group, (meth) acrylate not having an acid group, hydroperoxide as a polymerization initiator, a thiourea derivative as a reducing agent, and a vanadium compound as a polymerization accelerator.

7. The dental adhesive set as claimed in claim 6, wherein the dental adhesive comprises:
7 to 60% by weight of the (meth)acrylate not having an acid group;
3 to 30% by weight of the (meth)acrylate having an acid group;
0.01 to 10% by weight of the hydroperoxide as a polymerization initiator;
0.01 to 10% by weight of the thiourea derivative as a reducing agent; and
0.001 to 5% by weight of the vanadium compound.

8. The dental adhesive set as claimed in claim 6 or 7, wherein the dental adhesive further comprises a filler.

9. The dental adhesive set as claimed in claim 8, wherein a blending ratio of the filler in the dental adhesive is 0.5 to 80% by weight.

10. The dental adhesive set as claimed in any one of claims 6 to 9, wherein the vanadium compound in the dental adhesive set is one or more kinds selected from vanadium acetylacetonate, vanadyl acetylacetonate, vanadyl stearate, vanadium naphthenate, and vanadium benzoyl acetonate.
